# EUROPÄISCHE PATENTSCHRIFT

(11) **EP 2 694 459 B1**
(45) Veröffentlichungstag und Bekanntmachung des Hinweises auf die Patenterteilung: **18.05.2016**
(21) Anmeldenummer: 12710710.0
(22) Anmeldetag: 21.03.2012
(51) Int. Cl.: C07C 5/333, C07C 11/08, C07C 11/12, C07C 11/167, C07C 11/21, C07C 13/20, C07C 13/275

(54) **VERFAHREN ZUR HERSTELLUNG VON 1-BUTEN UND EINEM 1,3-BUTADIENDERIVAT**
PROCESS FOR PREPARING 1-BUTENE AND A 1,3-BUTADIENE DERIVATIVE
PROCÉDÉ DE PRÉPARATION DE 1-BUTÈNE ET D'UN DÉRIVÉE DE 1,3-BUTADIÈNE

(30) Priorität: 04.04.2011 DE 102011006721
(43) Veröffentlichungstag der Anmeldung: 12.02.2014
(73) Patentinhaber: Evonik Degussa GmbH, 45128 Essen (DE)
(72) Erfinder: MASCHMEYER, Dietrich, 45657 Recklinghausen (DE)
(86) Internationale Anmeldenummer: PCT/EP2012/054975
(87) Internationale Veröffentlichungsnummer: WO 2012/136479

(56) Entgegenhaltungen:
- WO-A1-2005/063658
- DE-A1- 10 231 633
- DE-A1- 10 350 045

## Beschreibung

Die vorliegende Erfindung betrifft ein Verfahren zur Herstellung von 1-Buten und einem 1,3-Butadienderivaten aus n-Butan oder einem Gemisch linearer C₄-Kohlenwasserstoffe, das n-Butan enthält.

1-Buten und Derivate des 1,3-Butadiens sind wichtige Zwischenprodukte für die Herstellung einer Vielzahl von Produkten. Beispielsweise kann 1-Buten zur Modifizierung von Ethylen- oder Propylenpolymeren verwendet werden. Das Butadienfolgeprodukt 1-Methoxy-2,7-octadien ist beispielsweise ein Zwischenprodukt für die Synthese von 1-Octen.

Ungesättigte C₄-Kohlenwasserstoffe können aus den C₄-Fraktionen von Crackern, wie beispielsweise Steamcrackern oder FC-Crackern, die zur Herstellung von Propylen und Ethylen betrieben werden, gewonnen werden. Beispielsweise kann aus der C₄-Fraktion eines Steamcrackers 1-Buten und 1,3-Butadien und aus dem C₄-Schnitt eines FC-Crackers 1-Buten abgetrennt werden. Die Mengen an C₄-Schnitten sind an der Produktion von Ethylen und Propylen gekoppelt und stehen nicht in ausreichendem Maße zur Verfügung.

Alternativ dazu können lineare ungesättigte C₄-Kohlenwasserstoffe durch Dehydrierung von n-Butan hergestellt werden. Dabei entsteht ein Reaktionsgemische, die nicht umgesetztes n-Butan, 1-Buten, die beiden 2-Butene und 1,3-Butadien enthalten.

In DE 103 50 045 wird ein Verfahren zur Gewinnung von 1-Buten aus n-Butan beschrieben. Dabei wird n-Butan dehydriert und nach Abtrennung von Nebenprodukten aus dem Dehydrierprodukt, die keine C₄-Kohlenwasserstoffe sind, wird das Butadien selektiv zu linearen Butenen hydriert. Aus dem Hydriergemisch wird 1-Buten destillativ abgetrennt und das verbleibende Gemisch, das vorwiegend aus 2-Butenen und n-Butan besteht wird in die Dehydrierstufe zurückgeführt.

DE 102 31 633 offenbart ein Verfahren zur Herstellung von 4-Vinylcyclohexen aus n-Butan. Dabei wird n-Butan dehydriert und nach Abtrennung von Nebenprodukten aus dem Dehydrierprodukt, die keine C₄-Kohlenwasserstoffe sind, wird das Butadien katalytisch zu 4-Vinylcyclohexen umgesetzt. Nach Abtrennung des 4-Vinylcyclohexens wird das restliche Kohlenwasserstoffgemisch, das die linearen Butene, n-Butan und gegebenenfalls Butadien enthält, in den Dehydrierreaktor zurückgeführt.

Beiden Verfahren ist gemeinsam, dass jeweils nur eine Komponente aus dem Dehydrierungsgemisch gewonnen wird.

Das bei der Dehydrierung von n-Butan anfallende Reaktionsgemisch enthält neben den linearen Butenen, n-Butan und 1,3-Butadien. Die Gewinnung von reinem 1-Buten und reinem 1,3-Butadien, das in weiteren Schritt zu Folgeprodukte umgesetzt werden kann, aus solchen Mischungen durch Destillation ist wegen deren geringen Siedepunktsdifferenz nicht wirtschaftlich. Ebenfalls ist die Abtrennung des 1,3-Butadiens durch Extraktions(destillation) aufwändig und teuer.

Die Aufgabe der vorliegenden Erfindung besteht darin, ein Verfahren bereitzustellen, das es ermöglicht, 1-Buten und ein Butadienderivat wirtschaftlich aus n-Butan herzustellen.

Diese Aufgabe wird durch das im Folgenden beschriebene Verfahren gelöst.

Verfahren zur Herstellung von 1-Buten und einem 1,3-Butadienderivat umfassend die Verfahrensschritte:
a) nicht-oxidative katalytische Dehydrierung eines Einsatzgasstroms, welcher n-Butan, Wasserstoff, andere leichtsiedende Nebenbestandteile, Hochsieder und optional Wasser umfasst, so dass ein Produktgemisch entsteht, das nicht umgesetztes n-Butan, 1-Buten, die beiden 2-Butene, 1,3-Butadien, Wasserstoff, andere leichtsiedende Nebenbestandteile, Hochsieder und optional Wasser umfasst, wobei der Einsatzgasstrom keine C₄-Isoverbindungen aufweist;
b) Abtrennung von Wasserstoff, anderen Leichtsiedern, Hochsiedern und falls vorhanden von Wasser, so dass ein Produktgemisch erhalten wird, das n-Butan, 1-Buten, die beiden Butene und 1,3-Butadien umfasst;
c) Umsetzung eines Teils des in Verfahrensschritt b) erhaltenen 1,3-Butadiens zu einem Derivat;
d) Abtrennung des im Verfahrensschritt c) erhaltenen 1,3-Butadienderivats;
e) Selektivhydrierung des in Verfahrensschritt c) nicht derivatisierten 1,3-Butadiens zu 1-Buten;
f) destillative Abtrennung von 1-Buten aus dem im Schritt e) erhaltenen
Kohlenwasserstoffstrom, so dass ein Reststrom verbleibt.

In einer Ausführungsform des Verfahrens wird der im Verfahrensschritt f) anfallende Reststrom ganz oder teilweise dem Einsatzgasstrom zugeführt, also zurück in die Dehydriereinheit, in der der Verfahrensschritt a) abläuft.

Die vorliegende Erfindung hat den Vorteil, dass aus n-Butan kostengünstig 1-Buten und ein Butadienderivat hergestellt werden kann. Dabei kann das Mengenverhältnis der beiden Zielprodukte durch Einstellen der Dehydrierbedingungen und des Butadienumsatzes variiert werden. Die spezielle Ausführung der Erfindung zeichnet sich darüber hinaus dadurch aus, dass ein sehr hoher Anteil der bei der Dehydrierung entstandenen Olefine in Wertprodukte umgewandelt wird, sodass nur eine geringe Menge an Butenen mit dem Rückführstrom in den Dehydrierungsreaktor eingebracht wird.

In einer Ausführungsform der Erfindung werden die im Reststrom vorhandenen linearen Butene vor der Zuführung zumindest teilweise umgesetzt, und die Umsatzprodukte vor der Zuführung aus dem Reststrom entfernt.

In einer Ausführungsform der Erfindung handelt es sich bei dieser Umsetzung um eine Oligomerisierung.

### Einsatzstoffe

Als Einsatzstoffe für das erfindungsgemäße Verfahren können die n-Butanfraktion von Feldbutanen, Gemische linearer C₄-Kohlenwasserstoffe, die bei der Aufarbeitung von C₄-Schnitten von Steamcrackern oder FC-Crackern anfallen oder andere Gemische linearer C₄-Kohlenwasserstoffe, die bei anderen technischen Prozessen entstehen, verwendet werden.

Als Feldbutane bezeichnet man die C₄-Fraktion der "feuchten" Anteile des Erdgases sowie der Erdöl-Begleitgase, die durch Abkühlung auf etwa -30 °C in flüssiger Form aus den Gasen abgetrennt werden. Durch Tieftemperaturdestillation gewinnt man daraus die Feldbutane, deren Zusammensetzung je nach Lagerstätte schwankt, die jedoch im Allgemeinen etwa 30 Massen-% Isobutan und 65 Massen-% n-Butan enthalten. Weitere Bestandteile sind in der Regel etwa 2 Massen-% Kohlenwasserstoffe mit weniger als 4 C-Atomen und etwa 3 Massen-% Kohlenwasserstoffe mit mehr als 4 C-Atomen. Dieses Gemisch kann nach destillativer Abtrennung des Isobutans im erfindungsgemäßen Verfahren eingesetzt werden. Optional werden vor dem Dehydrierungsschritt des erfindungsgemäßen Verfahrens auch die Kohlenwasserstoffe, die keine 4 C-Atome aufweisen, ganz oder teilweise abgetrennt.

In einer Ausführungsform des Verfahrens ist der Einsatzgasstrom aus dem Verfahrensschritt a) die n-Butanfraktion von Feldbutanen.

In einer weiteren Ausführungsform des Verfahrens ist der Einsatzgasstrom aus dem Verfahrensschritt a) ein Gemisch linearer C₄-Kohlenwasserstoffe aus der Aufarbeitung von C₄-Schnitten von Steamcrackern oder FC-Crackern.

### Derivatisierung des 1,3-Butadiens

Das nach Abtrennung der Nebenprodukte erhaltene Kohlenwasserstoffgemisch umfasst im Wesentlichen n-Butan, 1-Buten, die beiden 2-Butene und 1,3-Butadien.

Dieses Gemisch wird einer Reaktion unterworfen, bei der das 1,3-Butadien, jedoch nicht die linearen Butenen umgesetzt werden.

In einer Ausführungsform des Verfahrens wird 1,3-Butadien im Verfahrensschritt c) zu einem Derivat umgesetzt ausgewählt aus: 4-Vinylcyclohexen, 1,4-Cyclooctadien, 1,5,9-Cyclododecatrien, 4-Cyclohexen-1,2-dicarbonsäurederivaten, 1,7-Octadien, unverzweigten acyclischen Octatrienen, 2,7-Octadienylderivaten.

Die Umsetzung von 1,3-Butadien zu 4-Vinylcyclohexen kann beispielsweise an Cu(I)-Trägerkatalysatoren, wie in US 5,196,621 oder nach EP 0 397 266 erfolgen.

1,3-Butadien kann in Gegenwart von gelösten Nickel-Alumo-organischen Katalysatoren zu 1,4-Cyclooctadien und/oder 1,5,9-Cyclododecatrien umgesetzt werden.

Die reduktive Dimerisierung von 1,3-Butadien zu 1,7-Octadien kann nach DE 101 49 347 bzw. DE 10 2006 031413.1 durchgeführt werden.

Die Dimerisierung von 1,3-Butadien zu Octatrien, insbesondere zu 1,3,7-Octatrien, kann an einem Palladiumcarbenkomplex durchgeführt werden, wie in DE 10 2004 060520 beschrieben.

In einer Ausführungsform des Verfahrens wird das 1,3-Butadien im Verfahrensschritt c) mit Dienophilen, die eine elektronenarme C-C-Mehrfachbindung aufweisen, zu Diels-Alder-Produkten umgesetzt. Die Mehrfachbindung kann eine C-C-Doppelbindung oder eine C-C-Dreifachbindung sein.

Dienophile mit Dreifachbindungen sind beispielsweise:
Propinsäure; Propinsäureester, wobei der am Sauerstoffatom des Esters gebundene Rest 1 bis 20 C-Atome aufweisen kann; Propinal; Propinol; Acetylendicarbonsäure; Acetylendicarbonsäuremonoester und Acetylendicarbonsäurediester, wobei der/die an einem Sauerstoffatom des Esters gebundene Rest 1 bis 20 C-Atome aufweisen kann/können; 3-Formylpropinsäure und ihre Ester; Butindial; Butindiol.

Dienophile mit Doppelbindungen weisen mindestens eine Doppelbindung auf, die mit einer oder mehreren elektronenziehenden Gruppe(n) substituiert und konjugiert ist. Entsprechende elekronenziehende Gruppen (-M-Effekt) sind: Nitrogruppe, Cyanogruppe, Formylrest, Ketorest (-C(O)R). Säurerest (-C(O)OH), Esterrest (-C(O)OR) oder Anhydridrest (-C(O)OC(O)R.

Es ist auch möglich, dass zwei vinciale Substituenten zusammen eine funktionale Gruppe bilden, beispielsweise eine Anhydridgruppe.

Bevorzugt eingesetzte Dienophile sind:
Maleinsäureanhydrid; Maleinsäure und ihre Alkylester, bei denen die Alkylreste gleich oder verschieden sein können und jeweils 1 bis 10 C-Atome, insbesondere 1 bis 4 C-Atome aufweisen; Fumarsäure und ihre Alkylester, bei denen die Alkylreste gleich oder verschieden sein können und jeweils 1 bis 10 C-Atome, insbesondere 1 bis 4 C-Atome aufweisen; Maleinsäureimid (Maleinimid) und seine N-substituierten Derivate, bei denen der Substituent am Stickstoff 1 bis 10, insbesondere 1 bis 4 C-Atome aufweist.

Dabei entstehen Derivate der 4-Cyclohexen-1,2-dicarbonsäure. Diese können beispielsweise durch Hydrierung der Doppelbindung und anschließender Alkoholyse (Veresterung, Umesterung) in Ester der 1,2-Cyclohexandicarbonsäure übergeführt werden. Diese Ester mit Esteralkylgruppen, die 7 bis 12 C-Atome aufweisen werden als Weichmacher verwendet, wie beispielsweise 1,2-Cyclohexandicarbonsäuredüsononylester.

In einer Ausführungsform des Verfahrens wird 1,3-Butadien mit einem protischen Nucleophil (Wasser, Alkohole, Amine) zu dem entsprechenden 2,7-Octadienylderivat umgesetzt, wobei der Nucleophilrest am C1 gebunden ist. Diese Umsetzung (Telomerisation) wird durch Palladiumkomplexe katalysiert. Vorzugsweise werden Palladiumcarbenkomplexe verwendet, wie beispielsweise in DE 101 28 144 und DE 103 12 829 beschrieben.

Die Durchführung der Telomerisation kann, ähnlich wie in DE 10 2005 036039 beschrieben, erfolgen, mit dem Unterschied, dass auf eine hydrierende Vorreinigung des Einsatzstroms verzichtet werden kann.

In einer Ausführungsform des Verfahrens wird als 2,7-Octadienylderivat 1-Methoxyocta-2,7-dien gebildet.

1-Methoxyocta-2,7-dien ist ein begehrtes Telomerisationsprodukt. Daraus kann durch Hydrierung der beiden olefinischen Doppelbindungen und anschließende Methanolabspaltung 1-Octen gewonnen werden, das zur Modifizierung von Polyethylen oder Polypropylen technisch eingesetzt wird. Die ausgehend von 1,3-Butadien dreistufige Synthese für 1-Octen ist beispielsweise in DE 101 49 348 veröffentlicht. Für die Methanolabspaltung aus 1-Methoxyoctan kann der in DE 102 57 499 beanspruchte Katalysator verwendet werden.

### Selektivhydrierung

Das nach der Abtrennung des Butadienderivats verbleibende C₄-Kohlenwasserstoffgemisch enthält neben nicht umgesetzten 1,3-Butadien 1-Buten und, wenn nicht bereits vorher abgetrennt, n-Butan und die beiden 2-Butene. Die Restmengen an 1.3-Butadien und gegebenenfalls vorhandenen mehrfach ungesättigte Kohlenwasserstoffe, wie beispielsweise 1,2-Butadien werden durch Selektivhydrierung entfernt, die zudem die Anteile an n-Butenen erhöht. Ein geeignetes Verfahren ist z. B. von F. Nierlich et al. In Erdöl & Kohle, Erdgas, Petrochemie, 1986, Seite 73 ff beschrieben. Es arbeitet in flüssiger Phase mit vollständig gelöstem Wasserstoff in stöchiometrischen Mengen. Als selektive Hydrierkatalysatoren eignen sich z. B. Nickel und insbesondere Palladium auf einem Träger, z. B. 0,3 Massen-% Palladium auf Aktivkohle oder Aluminiumoxid. Eine geringe Menge Kohlenmonoxid im ppm-Bereich fördert die Selektivität der Hydrierung des 1,3-Butadiens zu den linearen Butenen und wirkt der Bildung von Polymeren, dem sogenannten "green oil" entgegen, die den Katalysator inaktivieren.

### Abtrennung des 1-Butens

Der Hydrieraustrag wird destillativ in 1-Buten und in ein Gemisch aus n-Butan und linearen Butenen, hauptsächlich 2-Butenen, getrennt.

### Verwendung der Destillatfraktionen

Das gewonnene 1-Buten enthält keine Isoverbindungen. Es kann insbesondere für die Herstellung von Cooligomeren mit Ethylen oder Propylen oder als Comonomer in Polyolefinen (LLDPE) verwendet werden.

In einer weiteren Ausführungsform des Verfahrens wird das im Verfahrensschritt f) gewonnene 1-Buten in einem anschließenden Verfahrenschritt g) zu Cooligomeren mit Ethylen oder Propylen umgesetzt.

Die n-Butan/2-Buten-Fraktion kann ganz oder teilweise in den Dehydrierreaktor zurückgefahren werden.

Optional kann vor der Rückführung ein Teil der linearen Butene durch Umsetzung und Abtrennung der Umsetzungsprodukte entfernt werden.

Geeignete Umsetzungen, die begehrte Zwischenprodukte liefern, sind beispielsweise die Oligomerisierung oder Hydroformylierung.

Die Oligomerisierung kann unter Verwendung von sauren oder Nickel enthaltenden Katalysatoren homogen oder heterogen durchgeführt werden. Bevorzugt erfolgt die Oligomerisierung an Nickel-Festbettkatalysatoren. Ein solches Verfahren ist beispielsweise das Octol-Verfahren der Evonik Oxeno GmbH. Die dabei hauptsächlich entstehenden Olefine mit 8 und 12 C-Atomen sind Zwischenprodukte für die Herstellung von Weichmachern oder Detergenzien.

Bei der Hydroformylierung entsteht ein Gemisch aus n-Pentanal und 2-Methylbutanal. Durch Wahl des verwendeten Katalysators kann das Massenverhältnis der beiden Aldehyde variiert werden. Unter isomerisierenden Bedingungen ist es möglich, n-Pentanal in über 95 %-iger Selektivität herzustellen. Dazu kann ein Katalysatorsystem eingesetzt werden, wie beispielsweise in EP 0 213 639 beschrieben. Solche Gemische sind insbesondere für die Herstellung von Decanolgemischen mit hohem Anteil von 2-Propylheptanol geeignet.

### Ausführungsbeispiel

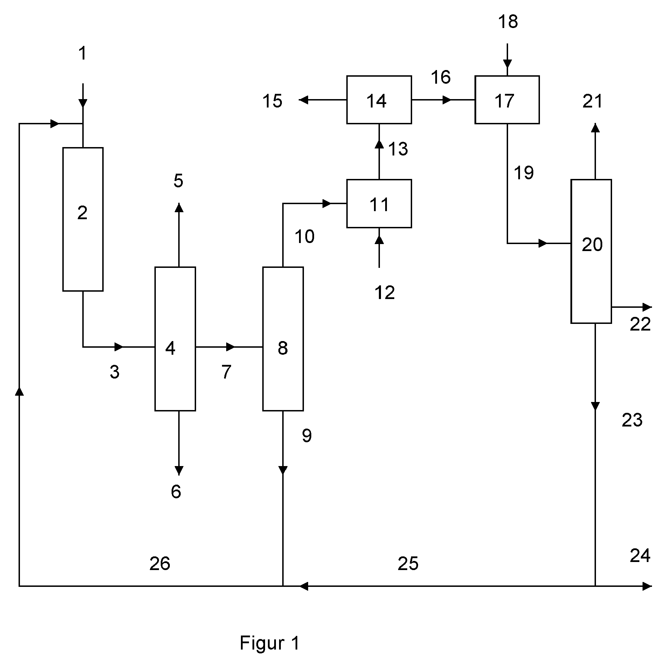

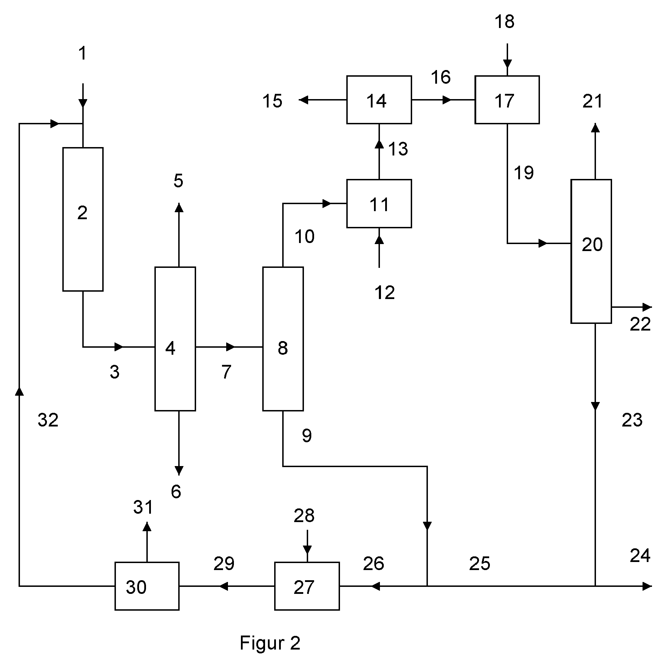

Zwei Ausführungsbeispiele der vorliegenden Erfindung werden durch die Blockschemata in den Figuren 1 und 2 erläutert.

Bei dem in Figur 1 dargestellten Ausführungsbeispiel wird der n-Butan enthaltende Einsatzstrom (1) zusammen mit dem Rückführstrom (25/26) in die Dehydriereinheit (2) eingeleitet (gegebenenfalls kann Wasserdampf oder Sauerstoff eingeleitet werden; dies ist in der Figur 1 nicht dargestellt). Das Dehydrierungsgemisch (3) wird in einer Destillationseinheit (4) in Leichtsieder (5), Hochsieder einschließlich Wasser (6) und eine C₄-Fraktion (7) getrennt. Aus Strom (7) wird ein Teil des n-Butans und der beiden 2-Butene abgetrennt Strom (9), das in den Dehydrierungsreaktor zurückgeführt wird. Ein Teil des 1,3-Butadiens im Kopfstrom (10) wird im Reaktor (11), gegebenenfalls unter Zusatz eines Agens (12), derivatisiert. Aus dem Reaktionsgemisch (13) werden in der Aufarbeitungsapparatur (14) das Butadienderivat (15), ein Zielprodukt, und die C₄-Fraktion (16) abgetrennt. Die Abtrennung von gegebenenfalls vorhandenen Agens, Katalysator und deren Zurückführung ist nicht dargestellt. Der C4-Strom (16), der noch geringe Mengen an 1,3-Butadien enthält, wird im Reaktor (17) mit Wasserstoff (18) selektiv hydriert. Der Hydrieraustrag (19) wird in der Hydriereinheit (20) in 1-Buten (21), zweites Zielprodukt (22), in ein Gemisch (23) aus n-Butan und linearen Butenen und gegebenenfalls eine Fraktion mit Hochsiedern getrennt. Der Strom (23) wird gegebenenfalls nach Entnahme eines Teilstroms (24) in den Dehydrierungsreaktor zurückgeführt.

Bei dieser Ausführungsform ist die Kolonne (8) optional. Deren Verwendung bietet den Vorteil, dass die Konzentration an 1,3-Butadien im Strom (10) erhöht wird. Dadurch kann im Reaktor (11) eine höhere Umsatzgeschwindigkeit für 1,3-Butadien erreicht werden. Nachteilig ist jedoch der Kapitaleinsatz für die Kolonne und deren Betriebskosten.

Eine zweite Ausführungsform der vorliegenden Erfindung wird in Figur 2 dargelegt. Sie unterscheidet sich von der Ausführungsform 1 dadurch, dass aus dem Rückstrom (26) ein Teil der linearen Butene im Reaktor (27), gegebenenfalls unter Zusatz eines Agens (28), zum Strom (29), bestehend aus n-Butan, nicht umgesetzten Butenen und dem Produkt der Reaktion, umgesetzt wird. Nach Abtrennung der Reaktionsprodukte (31) und gegebenenfalls anderer Stoffe in der Trennapparatur (30) wird Strom (32), der n-Butan und lineare Butene enthält, in den Dehydrierungsreaktor eingespeist.

Bei dieser Ausführungsform ist es optional, nur Strom (9) oder nur Strom (23) oder Teile dieser beiden Ströme im beliebigen Verhältnis in den Reaktor einzuspeisen.

## Patentansprüche

1. Verfahren zur Herstellung von 1-Buten und einem 1,3-Butadienderivat, umfassend die Verfahrensschritte:
a) nicht-oxidative katalytische Dehydrierung eines Einsatzgasstroms, welcher n-Butan, Wasserstoff, andere leichtsiedende Nebenbestandteile und Hochsieder umfasst, so dass ein Produktgemisch entsteht, das nicht umgesetztes n-Butan, 1-Buten, die beiden 2-Butene, 1,3-Butadien, Wasserstoff, andere leichtsiedende Nebenbestandteile und Hochsieder umfasst;
b) Abtrennung von Wasserstoff, anderen Leichtsiedern und Hochsiedern, so dass ein Produktgemisch erhalten wird, das n-Butan, 1-Buten, die beiden Butenen und 1,3-Butadien umfasst;
c) Umsetzung eines Teils des in Verfahrensschritt b) erhaltenen 1,3-Butadiens zu einem Derivat;
d) Abtrennung des im Verfahrensschritt c) erhaltenen 1,3-Butadienderivats;
e) Selektivhydrierung des in Verfahrensschritt c) nicht derivatisierten 1,3-Butadiens zu 1-Buten;
f) destillative Abtrennung von 1-Buten aus dem im Schritt e) erhaltenen Kohlenwasserstoffstrom, so dass ein Reststrom verbleibt.

2. Verfahren nach Anspruch 1,
wobei der im Verfahrensschritt f) anfallende Reststrom ganz oder teilweise dem Einsatzgasstrom zugeführt wird.

3. Verfahren nach Anspruch 2,
wobei die im Reststrom vorhandenen linearen Butene vor der Zuführung zumindest teilweise umgesetzt werden, und die Umsatzprodukte vor der Zuführung aus dem Reststrom entfernt werden.

4. Verfahren nach Anspruch 3,
wobei es sich bei der Umsetzung um eine Oligomerisierung handelt.

5. Verfahren nach einem der Ansprüche 1 bis 4,
wobei der Einsatzgasstrom aus dem Verfahrensschritt a) die n-Butanfraktion von Feldbutanen ist.

6. Verfahren nach einem der Ansprüche 1 bis 4,
wobei der Einsatzgasstrom aus dem Verfahrensschritt a) ein Gemisch linearer C₄-Kohlenwasserstoffe aus der Aufarbeitung von C₄-Schnitten von Steamcrackern oder FC-Crackern ist.

7. Verfahren nach einem der Ansprüche 1 bis 6,
wobei 1,3-Butadien im Verfahrensschritt c) zu einem Derivat umgesetzt wird ausgewählt aus: 4-Vinylcyclohexen, 1,4-Cyclooctadien, 1,5,9-Cyclododecatrien, 4-Cyclohexen-1,2-dicarbonsäurederivaten, 1,7-Octadien, unverzweigten acyclischen Octatrienen, 2,7-Octadienylverbindungen.

8. Verfahren nach einem der Ansprüche 1 bis 6,
wobei das 1,3-Butadien im Verfahrensschritt c) mit Dienophilen, die eine elektronenarme C-C-Mehrfachbindung aufweisen, zu Diels-Alder-Produkten umgesetzt wird.

9. Verfahren nach Anspruch 8,
wobei das Dienophil ausgewählt ist aus:
- Maleinsäureanhydrid,
- Maleinsäure und ihre Alkylester, bei denen die Alkylreste gleich oder verschieden sein können und jeweils 1 bis 10 C-Atome aufweisen,
- Fumarsäure und ihre Alkylester, bei denen die Alkylreste gleich oder verschieden sein können und jeweils 1 bis 10 C-Atome aufweisen,
- Maleinsäureimid und seine N-substituierten Derivate, bei denen der Substituent am Stickstoff 1 bis 10 C-Atome aufweist.

10. Verfahren nach einem der Ansprüche 1 bis 6,
wobei das 1,3-Butadien im Verfahrensschritt c) mit einem protischen Nucleophil zu dem entsprechenden 2,7-Octadienylderivat umgesetzt wird, und wobei der Nucleophilrest am C1 gebunden ist.

11. Verfahren nach Anspruch 10,
wobei als 2,7-Octadienylverbindung 1-Methoxyocta-2,7-dien gebildet wird.

12. Verfahren nach einem der Ansprüche 1 bis 11,
wobei die Selektivhydrierung im Verfahrensschritt e) unter Einsatz eines Palladium-Katalysators erfolgt.

13. Verfahren nach Anspruch 12,
wobei der Palladium-Katalysators auf einem Träger aufgebracht ist ausgewählt aus:
Aktivkohle, Aluminiumoxid.

14. Verfahren nach einem der Ansprüche 1 bis 13,
wobei das im Verfahrensschritt f) gewonnene 1-Buten in einem anschließenden Verfahrenschritt g) zu Cooligomeren mit Ethylen oder Propylen umgesetzt wird.

## Claims

1. Process for preparing 1-butene and a 1,3-butadiene derivative, comprising the steps of:
a) non-oxidatively catalytically dehydrogenating a feedstock gas stream comprising n-butane, hydrogen, other low-boiling secondary constituents and high boilers, to form a product mixture comprising unreacted n-butane, 1-butene, the two 2-butenes, 1,3-butadiene, hydrogen, other low-boiling secondary constituents and high boilers;
b) removing hydrogen, other low boilers and high boilers, to give a product mixture comprising n-butane, 1-butene, the two butenes and 1,3-butadiene;
c) reacting some of the 1,3-butadiene obtained in step b), to form a derivative;
d) removing the 1,3-butadiene derivative obtained in step c);
e) selectively hydrogenating the 1,3-butadiene not derivatized in step c), to form 1-butene;
f) distillatively removing 1-butene from the hydrocarbon stream obtained in step e), to leave a residual stream.

2. Process according to Claim 1,
wherein the residual stream obtained in step f) is supplied wholly or partly to the feedstock gas stream.

3. Process according to Claim 2,
wherein the linear butenes present in the residual stream are at least partly reacted before the supplying, and the reaction products are removed from the residual stream before the supplying.

4. Process according to Claim 3,
wherein the reaction is an oligomerization.

5. Process according to any of Claims 1 to 4,
wherein the feedstock gas stream from step a) is the n-butane fraction of field butanes.

6. Process according to any of Claims 1 to 4,
wherein the feedstock gas stream from step a) is a mixture of linear C₄ hydrocarbons from the processing of C₄ cuts from steam crackers or FC crackers.

7. Process according to any of Claims 1 to 6,
wherein 1,3-butadiene is reacted in step c) to form a derivative selected from the following: 4-vinylcyclohexene, 1,4-cyclooctadiene, 1,5,9-cyclododecatriene, 4-cyclohexene-l,2-dicarboxylic acid derivatives, 1,7-octadiene, unbranched acyclic octatrienes, 2,7-octadienyl compounds.

8. Process according to any of Claims 1 to 6,
wherein the 1,3-butadiene is reacted in step c) with dienophiles which have an electron-deficient C-C multiple bond, to form Diels-Alder products.

9. Process according to Claim 8,
wherein the dienophile is selected from the following:
- maleic anhydride,
- maleic acid and its alkyl esters in which the alkyl radicals may be identical or different and each have 1 to 10 C atoms,
- fumaric acid and its alkyl esters in which the alkyl radicals may be identical or different and each have 1 to 10 C atoms,
- maleimide and its N-substituted derivatives in which the substituent on the nitrogen has 1 to 10 C atoms.

10. Process according to any of Claims 1 to 6,
wherein the 1,3-butadiene is reacted in step c) with a protic nucleophile to form the corresponding 2,7-octadienyl derivative, and wherein the nucleophile residue is attached to the C1.

11. Process according to Claim 10,
wherein said 2,7-octadienyl compound formed is 1-methoxyocta-2,7-diene.

12. Process according to any of Claims 1 to 11,
wherein the selective hydrogenation in step e) takes place using a palladium catalyst.

13. Process according to Claim 12,
wherein the palladium catalyst is applied on a support selected from the following: activated carbon, aluminium oxide.

14. Process according to any of Claims 1 to 13,
wherein the 1-butene recovered in step f) is reacted in a subsequent step g) to form cooligomers with ethylene or propylene.

## Revendications

1. Procédé de fabrication de 1-butène et d'un dérivé de 1,3-butadiène, comprenant les étapes de procédé :
a) la déshydrogénation catalytique non oxydative d'un courant gazeux d'entrée, qui comprend du n-butane, de l'hydrogène, d'autres constituants secondaires de point d'ébullition faible et des composants de point d'ébullition élevé, de manière à former un mélange de produits, qui comprend du n-butane non réagi, du 1-butène, les deux 2-butènes, du 1,3-butadiène, de l'hydrogène, d'autres constituants secondaires de point d'ébullition faible et des composants de point d'ébullition élevé ;
b) la séparation de l'hydrogène, des autres composants de point d'ébullition faible et des composants de point d'ébullition élevé, de manière à obtenir un mélange de produits, qui comprend du n-butane, du 1-butène, les deux butènes et du 1,3-butadiène ;
c) la transformation d'une partie du 1,3-butadiène obtenu à l'étape de procédé b) en un dérivé ;
d) la séparation du dérivé de 1,3-butadiène obtenu à l'étape de procédé c) ;
e) l'hydrogénation sélective du 1,3-butadiène non dérivé à l'étape de procédé c) en 1-butène ;
f) la séparation par distillation du 1-butène du courant d'hydrocarbures obtenu à l'étape e), de sorte qu'un courant résiduel demeure.

2. Procédé selon la revendication 1, dans lequel le courant résiduel formé à l'étape de procédé f) est introduit en totalité ou en partie dans le courant gazeux d'entrée.

3. Procédé selon la revendication 2, dans lequel les butènes linéaires présents dans le courant résiduel sont au moins partiellement transformés avant l'introduction et les produits transformés sont éliminés du courant résiduel avant l'introduction.

4. Procédé selon la revendication 3, dans lequel la réaction est une oligomérisation.

5. Procédé selon l'une quelconque des revendications 1 à 4, dans lequel le courant gazeux d'entrée de l'étape de procédé a) est la fraction de n-butane de butanes de champ.

6. Procédé selon l'une quelconque des revendications 1 à 4, dans lequel le courant gazeux d'entrée de l'étape de procédé a) est un mélange d'hydrocarbures en C₄ linéaires issus du traitement de coupes en C₄ de vapocraqueurs ou de craqueurs FC.

7. Procédé selon l'une quelconque des revendications 1 à 6, dans lequel le 1,3-butadiène est transformé à l'étape de procédé c) en un dérivé choisi parmi : le 4-vinylcyclohexène, le 1,4-cyclooctadiène, le 1,5,9-cyclododécatriène, les dérivés de l'acide 4-cyclohexène-1,2-dicarboxylique, le 1,7-octadiène, les octatriènes acycliques non ramifiés, les composés de 2,7-octadiényle.

8. Procédé selon l'une quelconque des revendications 1 à 6, dans lequel le 1,3-butadiène est mis en réaction à l'étape de procédé c) avec des diénophiles, qui comprennent une liaison C-C multiple pauvre en électrons, pour former des produits de Diels-Alder.

9. Procédé selon la revendication 8, dans lequel le diénophile est choisi parmi :
- l'anhydride de l'acide maléique,
- l'acide maléique et ses esters alkyliques, dans lesquels les radicaux alkyle peuvent être identiques ou différents et comprennent à chaque fois 1 à 10 atomes C,
- l'acide fumarique et ses esters alkyliques, dans lesquels les radicaux alkyle peuvent être identiques ou différents et comprennent à chaque fois 1 à 10 atomes C,
- l'imide de l'acide maléique et ses dérivés N-substitués, dans lesquels le substituant sur l'azote comprend 1 à 10 atomes C.

10. Procédé selon l'une quelconque des revendications 1 à 6, dans lequel le 1,3-butadiène est mis en réaction à l'étape de procédé c) avec un nucléophile protique pour former le dérivé de 2,7-octadiényle correspondant, et le radical nucléophile est relié à C1.

11. Procédé selon la revendication 10, dans lequel le 1-méthoxyocta-2,7-diène est formé en tant que composé de 2,7-octadiényle.

12. Procédé selon l'une quelconque des revendications 1 à 11, dans lequel l'hydrogénation sélective à l'étape de procédé e) a lieu en utilisant un catalyseur de palladium.

13. Procédé selon la revendication 12, dans lequel le catalyseur de palladium est appliqué sur un support choisi parmi : le charbon actif, l'oxyde d'aluminium.

14. Procédé selon l'une quelconque des revendications 1 à 13, dans lequel le 1-butène obtenu à l'étape de procédé f) est transformé lors d'une étape de procédé g) ultérieure en co-oligomères avec de l'éthylène ou du propylène.
